# EUROPEAN PATENT APPLICATION

(11) **EP 1 908 455 A1**
(43) Date of publication of application: **09.04.2008**
(21) Application number: 06711918.0
(22) Date of filing: 18.01.2006
(51) Int. Cl.: A61K 8/84, A61K 8/64, A61Q 5/00, A61Q 7/00

(54) **HAIR CARE PREPARATION**

(30) Priority: 13.06.2005 JP 2005172806
(71) Applicant: Nissei Bio Co., Ltd., Eniwa-shi Hokkaido 061-1374 (JP)
(72) Inventor: MATSUNAGA, Masaji, 1300012 (JP)
(74) Representative: Scott, Susan Margaret
(86) International application number: PCT/JP2006/300649
(87) International publication number: WO 2006/134685

(57) **Abstract**

It is intended to provide a hair care preparation. The invention is directed to a hair care preparation (a hair regrowth agent, a hair restorer or an agent for preventing hair loss) comprising as an effective component, an enzymatic or hydrolytic degradation product of a nucleoprotein and/or DNA or RNA, a deoxyoligonucleotide, a deoxymononucleotide, an oligopeptide, an oligonucleotide or a mononucleotide spearated from the degradation product, or a mixture of at least two members selected from the degradation products or compounds. The effective component such as a deoxyloligonucleotide has a relatively small molecular weight, therefore, it is easy to be percutaneously absorbed. Further it has a cell activation effect and a blood circulation-promoting effect when it is percutaneously absorbed. Therefore, when it is applied to the scalp, an excellent hair regrowth-promoting effect, hair restoration effect and effect of preventing hair loss are exhibited. The hair care preparation of the present invention is useful as a hair care pharmaceutical product, quasi-drug or cosmetic product for mammal, particularly for humans, or a preparation to be added thereto.

## Description

### [Technical Field]

The present invention relates to a hair care preparation, further specifically to a hair regrowth agent, a hair restorer and an agent for preventing hair loss containing, as effective component, an enzymatic or hydrolytic degradation product of nucleoprotein and/or DNA or RNA, or a deoxyoligonucleotide, a deoxymononucleotide, an oligopeptide, an oligonucleotide and a mononucleotide separated from the degradation product, or a mixture of at least two members selected from the degradation product or the compounds.

### [Background Art]

It is thought that hair loss, particularly, hair loss of male in or after middle age is caused by various matters such as an effect that male hormone secreted in a body is activated at hair follicle and sebaceous gland, an adverse effect on scalp due to increased peroxide concentration in a body, excess secretion of head sebum, reduction of blood flow volume to hair roots, malnutrition, and mental stress.
When head hair becomes thin by hair loss, generally, people tend to apparently give others an impression of higher age than the real age, and they tend to think themselves as being aged abruptly. Hence, there are many people who wish to keep thick head hair as long as possible, or to recover the thin head hair to the thick state, accordingly, there are strong general concerns about hair regrowth agents, hair restorers and agents for preventing hair loss.

In regard to studies on conventional hair care preparations (hair regrowth agent, hair restorer or agent for preventing hair loss), there have been done many attempts of hair regrowth and hair restoration as well as hair loss is prevented by blending a component for removing the cause of hair loss into a hair care preparation.
However, conventional hair care preparations have not yet reached a stage exhibiting prevention of hair loss and, hair regrowth and hair restoration-promoting effects sufficiently. This fact is thought as follows: causes of hair loss, hair regrowth and hair restoration are complicate, and combination of various causes appears as phenomena of hair loss, hair regrowth and hair restoration. Therefore, it has been desired to develop a new hair care preparation with high effects of prevention of hair loss, hair regrowth and hair restoration.

Incidentally, being reflected in the enhancement of general concerns about health in recent years, healthy foods using deoxyribonucleic acid (DNA), ribonucleic acid (RNA) or nucleoprotein as a raw material or an effective component have been provided. To dissolve a high molecular weight nucleoprotein in water and easily digest it, a proposal for reducing the molecular weight has been made (Patent Document 1).
It has been known that deoxyribonucleic acid (DNA), ribonucleic acid (RNA) or nucleoprotein has antiaging effects, but there has been no sufficient approach of its application to a hair care preparation such as a hair regrowth agent, a hair restorer or an agent for preventing hair loss.
Patent Document 1: Japanese Patent Application Laid-open No. 2004-16143

### [Disclosure of the Invention]

### [Problems to be solved by the Invention]

There are various causes for hair loss, for example, poor circulation of blood in scalp, stress and the like, above all, the direct cause is that activity of hair mother cell is lowered and entered in a resting phase. Hence, as a hair care preparation applied in order to prevent hair loss effectively, and simultaneously to conduct hair regrowth and hair restoration, there is desired one which exhibits a cell activity to activate hair mother cells, and prevention of hair loss and, hair regrowth and hair restoration effects through promotion of circulation of blood, however, the prevention of hair loss and, hair regrowth and hair restoration effects of conventional hair care preparations are not sufficient yet.

The present inventors have keenly studied to obtain a hair care preparation having superior effects in prevention of hair loss and, hair regrowth and hair restoration effects to those of a conventional hair care preparation, as a result, have found that the excellent prevention of hair loss and, hair regrowth and hair restoration effects are present in a degradation product containing a deoxyoligonucleotide, a deoxymononucleotide or an oligopeptide which is obtained by an enzymatic or hydrolytic treatment of a nucleoprotein and/or DNA, or an oligonucleotide or a mononucleotide which is obtained by an enzymatic or hydrolytic treatment of RNA, or a mixture thereof, and completed the present invention.

### [Means to solve the Problems]

Namely, a hair regrowth agent of the present invention is characterized by containing, as an effective component, a degradation product obtained by an enzymatic or hydrolytic treatment of a nucleoprotein and/or DNA, or a deoxyoligonucleotide, a deoxymononucleotide or an oligopeptide separated from the degradation product; or a degradation product obtained by an enzymatic or hydrolytic treatment of RNA, or an oligonucleotide or a mononucleotide separated from the degradation product; or a mixture of at least two members selected from the degradation product of a nucleoprotein and/or DNA, the degradation product of RNA, a deoxyoligonucleotide, a deoxymononucleotide, an oligopeptide, an oligonucleotide and a mononucleotide.

In a preferred embodiment of the hair regrowth agent of the present invention, the nucleoprotein and DNA are preferably obtained frommilt of fish, and the fish is particularly preferably selected from the group consisting of salmon, trout, herring and cod.
Further, the RNA is preferably obtained from beer yeast.

Further, a hair restorer of the present invention is characterized by containing, as an effective component, a degradation product obtained by an enzymatic or hydrolytic treatment of a nucleoprotein and/or DNA, or a deoxyoligonucleotide, a deoxymononucleotide or an oligopeptide separated from the degradation product; or a degradation product obtained by an enzymatic or hydrolytic treatment of RNA, or an oligonucleotide or a mononucleotide separated from the degradation product; or a mixture of at least two members selected from the degradation product of a nucleoprotein and/or DNA, the degradation product of RNA, a deoxyoligonucleotide, a deoxymononucleotide, an oligopeptide, an oligonucleotide and a mononucleotide.

In a preferred embodiment of the hair restorer of the present invention, the nucleoprotein and DNA are preferably obtained from milt of fish, and the fish is particularly preferably selected from the group consisting of salmon, trout, herring and cod.
Further, the RNA is preferably obtained from beer yeast.

Further, an agent for preventing hair loss of the present invention is characterized by containing, as an effective component, a degradation product obtained by an enzymatic or hydrolytic treatment of a nucleoprotein and/or DNA, or a deoxyoligonucleotide, a deoxymononucleotide or an oligopeptide separated from the degradation product; or a degradation product obtained by an enzymatic or hydrolytic treatment of RNA, or an oligonucleotide or a mononucleotide separated from the degradation product; or a mixture of at least two members selected from the degradation product of a nucleoprotein and/or DNA, the degradation product of RNA, a deoxyoligonucleotide, a deoxymononucleotide, an oligopeptide, an oligonucleotide and a mononucleotide.

In a preferred embodiment of the agent for preventing hair loss of the present invention, the nucleoprotein and DNA are preferably obtained from milt of fish, and the fish is particularly preferably selected from the group consisting of salmon, trout, herring and cod.
Further, the RNA is preferably obtained from beer yeast.

### [Effects of the Invention]

The hair regrowth agent of the present invention contains, as an effective component, an enzymatic or hydrolytic degradation product of nucleoprotein and/or DNA or RNA, or a deoxyoligonucleotide, a deoxymononucleotide, an oligopeptide, an oligonucleotide and a mononucleotide separated from the degradation product, or a mixture of at least two members selected from the degradation product or the compounds. The deoxyoligonucleotide and so forth have a relatively small molecular weight, hence, it is percutaneously absorbed easily, or when it is percutaneously absorbed, it has a cell activation effect and a blood circulation-promoting effect. Therefore, when the hair regrowth agent of the present invention is applied to scalp, an excellent hair regrowth-promoting effect is exhibited.
The hair restorer of the present invention contains the same effective component as the hair regrowth agent of the present invention, hence, an excellent hair restoration-promoting effect is exhibited from the same reason.
The agent for preventing hair loss of the present invention contains the same effective component as the hair regrowth agent of the present invention, hence, an excellent hair loss prevention effect is exhibited from the same reason.
The hair care preparation of the present invention is useful as a hair care pharmaceutical product, quasi-drug or cosmetic product for mammals, particularly for humans, or a preparation to be added thereto.

### [Best Mode for Carrying Out the Invention]

As the effective component of a hair regrowth agent, a hair restorer and an agent for preventing hair loss in the present invention, being used as a purified product, a deoxyoligonucleotide or a deoxymononucleotide can be obtained by an enzymatic or hydrolytic treatment of DNA; an oligonucleotide or a mononucleotide can be obtained by an enzymatic or hydrolytic treatment of RNA; and oligopeptide can be obtained by an enzymatic or hydrolytic treatment of a nucleoprotein, respectively.

DNA and nucleoprotein can be obtained by extraction from milt of fish and purification thereof for example. The fish, for example, is salmon, trout, herring and cod, above all, salmon is preferable.

Hereinafter, DNA is described further in detail.
DNA as a raw material for producing a hair regrowth agent, a hair restorer or an agent for preventing hair loss in the present invention may be various conformations, for example, may be a double stranded, single stranded or circular DNA. Resources of DNA are various living things such as animals, plants and microbes. Testis (milt) of fish, which is waste in fish processing, of salmon, trout, herring and cod in particular, contains especially much DNA, but has not been utilized effectively as resource to date and most of the testis is discarded. Hence, from the viewpoint of conversion of waste to resource as well, it is desirable to utilize the DNA derived from the testis. Further, it is possible to use DNAobtained from thymus glands of mammals and birds such as a cow, a pig and a chicken. Moreover, synthesized DNA can also be used.

Additionally, to obtain DNA from fish milt, an extraction and purification method described in Japanese Patent Application Laid-open No. 2005-245394 can be used.
Concretely, fish milt is roughly crushed first, the roughly crushed fish milt is subjected to a proteolytic enzyme (protease) treatment under a condition of not degrading DNA, and the enzyme-treated solution is filtered. Then, the filtrate is dialyzed using a hollow fiber membrane having a molecular weight cutoff of 2, 000 to 1, 000, 000, degraded proteins and ions are removed and double stranded DNA is concentrated. Further, by getting precipitation as a double stranded DNA salt from a solution dialyzed, or by concentrating the solution, the precipitate or the concentrate is recovered.
The DNA salt obtained by the above-described method was dried to give a powder DNA salt and the powder DNA can be used as a raw material for producing the hair care preparation composition of the present invention.

RNA can be obtained by extracting from beer yeast followed by purification of the extract.

Enzymes to treat DNA and RNA are nuclease for example, above all, nuclease derived from blue mould is preferred.

An oligopeptide is obtained by hydrolyzing a nucleoprotein contained in fish milt with protease and nuclease.

The above-described protease is mainly consisted of trypsin. Trypsin is serine protease having a high specificity, and selectively hydrolyzes a peptide linkage at a carboxyl side of arginine and lysine, so that it is suitable to hydrolyze protamine containing a lot of arginine. Further, the protease can contain other protease, for example, chymotrypsin and the like in addition to trypsin..As a good protease, protease manufactured by Novozymes Japan Co., Ltd. (former NovoNordisk Bio Industries Co., Ltd.) can be mentioned.

The above-described nuclease hydrolyzes 3',5'-phosphodiester linkage of deoxyribonucleic acid (DNA) and ribonucleic acid (RNA) to generate 5'-(deoxy)nucleotide oligomer. Properties of the nuclease are not particularly limited, but it is preferable to have somewhat heat stability. Such nuclease can be available as a commercial product from Amano Enzyme Inc. (former Amano Pharmaceutical Co., Ltd.) and Sigma Corporation.

The important factor in a hydrolytic treatment of DNA, RNA and nucleoprotein using the above-described nuclease is a temperature for reaction. The reaction temperature must be in a range of 60 to 75°C, and 70°C is most preferable. When a reaction is conducted below the temperature range, conversion of DNA, RNA and nucleoprotein to a low molecule does not proceed sufficiently, which does not make the degradation product soluble in water. On the other hand, when a reaction is conducted at higher temperatures than the temperature range, conversion to a low molecule proceeds extremely, and thus, there is a fear that excellent effects of nucleoprotein are lost.

As described above, by treating DNA, RNA and nucleoprotein in a hydrolysis at 60 to 75°C using nuclease, it is possible to conduct the conversion into a low molecule for fraction of 1000 to 3000 in molecular weight to be contained as high as about 50 to 70%, thereby to produce a degradation product of DNA, RNA and nucleoprotein having solubility in water and percutaneous absorbability.
Additionally, to obtain an oligopeptide by a hydrolytic treatment of nucleoprotein, protease treatment and nuclease treatment are conducted, but the order of these treatments is not particularly limited. It is preferably treated first with protease, subsequently treated with nuclease, which is desirable from the viewpoints of schedule in operation and quality of the final product obtained.

As the effective component of a hair regrowth agent, a hair restorer and an agent for preventing hair loss in the present invention, a degradation product obtained by an enzymatic or hydrolytic treatment of a nucleoprotein and/or DNA, or a degradation product obtained by an enzymatic or hydrolytic treatment of RNA can be used as it is (without purification). The above-described degradation product may contain, for example, an amino acid or the like.
Further, as the effective component of a hair regrowth agent, a hair restorer and an agent for preventing hair loss in the present invention, from the degradation product of a nucleoprotein and/or DNA, or RNA, the following compounds separated and purified by using a common separation means and/or purification means: a deoxyoligonucleotide, a deoxymononucleotide, an oligopeptide, an oligonucleotide and a mononucleotide can be used.
In this case, a deoxyoligonucleotide and an oligopeptide which are contained in a degradation product of nucleoprotein and/or DNA and RNA, or which are separated and purified from the degradation product by a common separation means and/or purification means have preferably a chain length of 2 to 12.

The above-described degradation product and compound may be used alone, or in a mixture of at least 2 members thereof.
In the case of using a mixture of the degradation product and compound, the mixing ratio can be arbitrarily selected.
In this case, as the degradation product and compound, it is particularly preferable that at least any one of the above-described deoxyoligonucleotide and oligonucleotide with a chain length of 2 to 12 is included, the sum of contents of the deoxyoligonucleotide and oligonucleotide with a chain length of 2 to 12 is 20% or more relative to the total quantity of the degradation product and compound.
Further, the above-described degradation product and compound may be used in combination with common additional components of a hair regrowth agent, a hair restorer and an agent for preventing hair loss at a predetermined ratio.

Concentration of the effective component (the above-described degradation product and/or compound) in a hair regrowth agent, a hair restorer and an agent for preventing hair loss of the present invention can be suitably selected.

The dosage form that can be adopted for the hair care preparation (hair regrowth agent, hair restorer and agent for preventing hair loss) of the present invention is not particularly limited as long as it can be the dosage form applied to skin, particularly to scalp, for example, liquid, emulsion, ointment gel, aerosol or the like can be suitably selected. Further, the form of the above-described hair care preparation can arbitrarily be suitably selected. For example, the form such as a hair liquid, hair tonic, hair cream, mousse and gel can be adopted, and also added to shampoo and rinse.

In the hair care preparation of the present invention, known components used in conventional hair care preparations can be blended in addition to the above-described effective components. For example, as an antibacterial agent, hinoki-tiol, hexachlorophene, benzalconium chloride, cetylpyridinium chloride, undecylenic acid, trichlorocarbanilide, bithionol and the like can be blended alone or in combination thereof.

Further, in the hair care preparation of the present invention, as a pharmaceutical ingredient, there can be blended alone or in combination of: nicotinic-acid amide, benzyl nicotinate, vitamin E or the derivatives thereof, for example, blood-vessel dilators such as vitamin E acetate, senburi essence, carpronium chloride and acethylcholine derivatives; skin function-sthenic agents such as cepharanthine; antiphlogistic agents such as glycyrrhetic acid or the derivatives thereof and LITHOSPERMI RADIX essence; female hormonal agents such as estradiol and estrone; amino acids such as serine, methionine and arginine; vitamins such as vitamin A, vitamin B₁, vitamin B₆, biotin and pantothenic acid or the derivatives thereof.

Further, in the hair care preparation of the present invention, if necessary, there can be blended alone or in combination of: agents such as salicylic acid, zinc or the derivatives thereof, lactic acid or the alkyl esters; refrigeratives such as menthol; organic acids such as citric acid; and agents and additives ordinarily used in hair care preparations, for example, an antiseptic agent, surfactant, dispersion stabilizer, thickening agent, pH adjusting agent and purified water.

### [Examples]

In Examples and Comparative Examples shown below, the present invention will be described concretely and further in detail. The following Examples are only for the explanation of the present invention, and the technical scope of the present invention should not be limited to these Examples.
Blending quantity in the following Examples and Comparative Examples is % by mass relative to the total quantity. Further, the quantity of prototype 1 and prototype 2 used in Examples (ones containing respective degradation products obtained by an enzymatic or hydrolytic treatment of a nucleoprotein and/or DNA, or RNA) is represented as a solid content.

### 1. Production of (deoxy)oligonucleotide

DNA derived from salmon milt was subjected to a limited degradation using nuclease admitted as a food additive [for example, enzymatic preparation: nuclease "Amano" manufactured by Amano Enzyme Inc. (former Amano Pharmaceutical Co., Ltd.)]. The produced deoxymononucleotide and deoxyoligonucleotide were analyzed with an electrophoresis apparatus to determine the optimum condition.
Concretely, to warm water adjusted at about 65°C, a powder form DNA-Na salt as a raw material was introduced, after stirring, further heated to 70°C, 0.25% of nuclease relative to the raw material was added thereto, followed by reaction for 3 hours. Next, the reaction mixture was heated at 85°C for 10 minutes to deactivate nuclease, and then centrifuged, and then a spray drying method was applied to the supernatant, thereby to obtain a dried powder (degradation product) containing deoxyoligonucleotide.

### 2. Analysis of (deoxy)oligonucleotide

To warm water adjusted at about 65°C, a powder form DNA-Na salt derived from salmon milt as a raw material was introduced, after stirring, further heated to 70°C, 0.05% of enzymatic preparation: nuclease "Amano" manufactured by Amano Enzyme Inc. (formerAmano Pharmaceutical Co. , Ltd.) relative to the rawmaterial was added thereto, followed by reaction for 3 hours to give a degradation product. Next, after the product was heated at 85°C for 10 minutes to deactivate nuclease, the resulting degradation product was analyzed by HPLC.

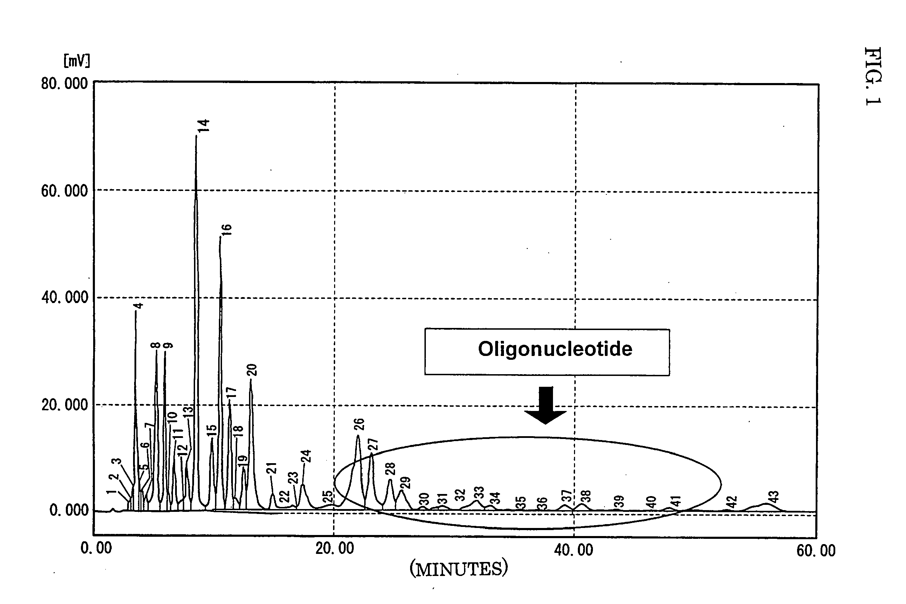

FIG. 1 shows an analysis example of deoxyoligonucleotide of the degradation product by HPLC (high-performance liquid chromatography). In FIG. 1, 5'-deoxymononucleotide and 3' -deoxymononucleotide solved out till a peak 20, relatively large peaks thereafter, that is, peaks after a peak 26 can be regarded as absorptions of deoxyoligonucleotide. Further, peaks after a peak 41 can be regarded as absorptions of the degradation products exceeding 3000 in molecular weight. Hence, on the basis of the result calculated from the peak intensities of the peak 26 to 41, it was understood that the present example contains a 31% fraction of deoxyoligonucleotide (molecular weight: 1000 to 3000) relative to the total of degradation products.

### 3. Production of hair care preparation (hair regrowth agent, hair restorer and agent for preventing hair loss) using (deoxy)oligonucleotide

The dried powder (degradation product) containing deoxyoligonucleotide obtained by the above-described production method is denoted as a prototype 1, using this prototype 1, the hair care preparation (hair regrowth agent, hair restorer and agent for preventing hair loss) of the present invention was produced as follows. As a reference, a hair care preparation containing no prototype 1 was also produced. These compositions are shown as a whole in the following Table 1.

### [Example 1]

Purified water was added to 95% ethanol, to which a hardened caster oil ethylene oxide (40 mol) adduct and stearyldimethylamine oxide were added and stirred, then, a prototype 1 was added thereto and dissolved while stirring to obtain a preparation of transparent liquid of Example 1.

### [Comparative Example 1]

A preparation of Comparative Example 1 was produced in the same method as Example 1 using an equal amount of glycerin instead of the prototype 1.

### 4. Measuring test of blood flow volume

The respective 10 µL preparations of Example 1 and Comparative Example 1 were applied to human upper arms, after one hour of the application, blood flow volume was measured with a laser Doppler tester. The evaluation of test results was conducted by the following criteria.
++: Blood flow volume was remarkably increased compared with Comparative Example (remarkably effective).
+ : Blood flow volume was increased compared with Comparative Example (effective).
± : Blood flow volume was somewhat increased compared with Comparative Example (somewhat effective).
- : Blood flow volume was equal to or less compared with Comparative Example (not effective).
   The results are shown as a whole in Table 1.

### 5. Hair regrowth test

Using mice corresponding to a resting phase in a hair regrowth and hair loss cycle as an experimental animal, the following test was conducted by setting each group to 10 mice. A mouse was shaved on the back with a shaving blade, the preparations of Examples 1 to 3 and the preparation of Comparative Example were applied to a predetermined area of the shaven region by 0.1 mL once every morning, the hair regrowth (regeneration) area was measured after 3 weeks, and the ratio of the hair regrowth area to the area that the preparation was applied was obtained in %.
The results are shown as a whole in the following Table 1.

### 6. Hair restoration promoting test

Hair restoration promoting effects were examined on the basis of hair root resting phase ratio (%). The preparations of Examples 1 to 3 and the preparation of Comparative Example were applied to men of human subject, that is, to 10 men in each group, twice a day by 4 mL each time on the scalp for 3 months. Hairs were removed from the top of head at random just before the start of test and after 3 month elapse, which were observed under a microscope to obtain the ratio of the resting phase from the condition of hair roots and, the test was evaluated by the following criteria based on the difference that the ratio of hair root resting phase ratio just before the start was subtracted from the ratio of hair root resting phase ratio after 3 month elapse.
++: Hair root resting phase ratio is reduced by at least 30% compared with Comparative Example
+ : Hair root resting phase ratio was reduced by at least 10% to less than 30% compared with Comparative Example
± : Hair root resting phase ratio was reduced by at least 0% to less than 10% compared with Comparative Example
- : Hair root resting phase ratio was reduced by less than 0% (increase) compared with Comparative Example
The results are shown as a whole in the following Table 1.

### 7. Hair loss prevention test

Hair loss prevention effect was examined by the change of the numbers of hair loss before and after use of sample due to shampoo. The study group was men of human subject, 10 men in each group, test period was 6 months, of which the first two month period was a period without application of sample and the latter four month period was a period with application of sample. During the sample application period, the preparations of Examples 1 to 3 and the preparation of Comparative Example were applied to the scalp twice a day by 4 mL each time for four months. In the test period, shampoo was done every other day and fallen hairs were collected, the number of hair loss was counted as a whole for one week. From this number, the number of hair loss per one shampoo (average value) was calculated, and the test was evaluated by the following criteria based on the comparison between the average value in the final week of the first period and the average value in the final week of the latter period.
++: Number of hair loss was decreased by at least 70, a remarkable hair loss prevention effect was observed.
+ : Number of hair loss was decreased by at least 40, not a little hair loss prevention effect was observed.
± : Number of hair loss was decreased by at least 10, a hair loss prevention effect was somewhat observed.
- : Number of hair loss was decreased by less than 10, or number of hair loss was increased, a hair loss prevention effect was not observed.
The results are shown as a whole in the following Table 1.

**Table 1**

| Composition of hair care preparation of Example 1 and Comparative Example 1, and Evaluation of test result | | |
|---|---|---|
| Blending component | Example 1 | Comparative Example 1 |
| Prototype 1 (DNA salt degradation product) | 1.0 | - |
| Glycerin | - | 1.0 |
| Hardened caster oil ethylene oxide (40 mol) adduct | 1.0 | 1.0 |
| Stearyldimethylamine oxide | 0.5 | 0.5 |
| 95% Ethanol | 54.0 | 54.0 |
| Purified water | Remainder | Remainder |
| Measuring test of blood flow volume | ++ | (Standard) |
| Hair regrowth test | 98% | 5% |
| Hair restoration promoting test | ++ | (Standard) |
| Hair loss prevention test | ++ | - |

### 8. Production of hair care preparation (hair regrowth agent, hair restorer and agent for preventing hair loss)

As an effective component, ones containing DNA, a nucleoprotein, and a degradation product obtained by an enzymatic or hydrolytic treatment of DNA and a nucleoprotein were denoted as a prototype 2 (one containing a deoxyoligonucleotide and a deoxymononucleotide), as a prototype 3 (one containing deoxyoligonucleotide, a deoxymononucleotide and an oligopeptide) and as a prototype 4 (one containing deoxyoligonucleotide, a deoxymononucleotide and an oligopeptide), respectively, and as an effective component, one containing a degradation product obtained by an enzymatic or hydrolytic treatment of RNA was denoted as a prototype 5 (one containing oligonucleotide and a mononucleotide).
Using the obtained prototypes 2 to 5, the hair care preparation (hair regrowth agent, hair restorer and agent for preventing hair loss) of the present invention was produced as follows. As a reference, the hair care preparation of Comparative Example 2 containing no prototypes 2 to 5 was also produced. The compositions of these preparations are shown as a whole in the following Table 2.

### [Examples 2 to 6]

To each prototype 2, prototype 3, prototype 4, prototype 5, and an equivalent mixture of prototype 2 and prototype 5 (prototype 6) were suitably added an antiseptic agent, surfactant, dispersion stabilizer, thickening agent and pH adjusting agent shown in the following Table 2, while stirring, these were dissolved in purified water to obtain preparations in a milky liquid form of Examples 2 to 6.

### [Comparative Example 2]

A preparation of Comparative Example 2 was obtained in the same way as Examples 2 to 6 using an equal amount of glycerin instead of the prototypes 2 to 6.

### 9. Performance test of hair care preparation (hair regrowth agent, hair restorer and agent for preventing hair loss)

In regard to the preparations of Examples 2 to 6 and Comparative Example 2, the above-described measuring test of blood flow volume, hair regrowh test, hair growth promoting test and hair loss prevention test were respectively conducted in the same conditions as in Example 1 and Comparative Example 1.
The results are shown as a whole in the following Table 2.

**Table 2**

| Composition of hair care preparation of Examples 1, 2, 3, and Comparative Example, and Evaluation of test result. | | | | | | |
|---|---|---|---|---|---|---|
| Blending component | Example 2 | Example 3 | Example 4 | Example 5 | Example 6 | Comparative Example 2 |
| Prototype 2 (DNA degradation product) | 1.0 | - | - | - | - | - |
| Prototype 3 (Nucleoprotein degradation product) | - | 1.0 | - | - | - | - |
| Prototype 4 (DNA + Nucleoprotein degradation product) | - | - | 1.0 | - | - | - |
| Prototype 5 (RNA degradation product) | - | - | - | 1.0 | 0.5 | - |
| Glycerin | - | - | - | - | - | 1.0 |
| Butylene glycol | Appropriate amount | Appropriate amount | Appropriate amount | Appropriate amount | Appropriate amount | Appropriate amount |
| Pentylene glycol | Appropriate amount | Appropriate amount | Appropriate amount | Appropriate amount | Appropriate amount | Appropriate amount |
| Phenoxyethanol | Appropriate amount | Appropriate amount | Appropriate amount | Appropriate amount | Appropriate amount | Appropriate amount |
| PEG-20 sorbitan cocoate | Appropriate amount | Appropriate amount | Appropriate amount | Appropriate amount | Appropriate amount | Appropriate amount |
| Sodium metaphosphate | Appropriate amount | Appropriate amount | Appropriate amount | Appropriate amount | Appropriate amount | Appropriate amount |
| Inositol | Appropriate amount | Appropriate amount | Appropriate amount | Appropriate amount | Appropriate amount | Appropriate amount |
| Carbomer | Appropriate amount | Appropriate amount | Appropriate amount | Appropriate amount | Appropriate amount | Appropriate amount |
| Potassium hydrate | Appropriate amount | Appropriate amount | Appropriate amount | Appropriate amount | Appropriate amount | Appropriate amount |
| Purified water | Remainder | Remainder | Remainder | Remainder | Remainder | Remainder |
| Measuring test of blood flow volume | ++ | ++ | ++ | ++ | ++ | (Standard) |
| Hair regrowth test | 98% | 96% | 97% | 95% | 97% | 5% |
| Hair restoration promoting test | ++ | ++ | ++ | ++ | ++ | (Standard) |
| Hair loss prevention test | ++ | ++ | ++ | ++ | ++ | - |

From the results shown in Table 1 and Table 2, the followings were found.
(1) The each evaluation of the preparations of Example 1 or Examples 2 to 6 by the measuring test of blood flow volume is ++ [Blood flow volume was remarkably increased compared with Comparative Example 1 or 2 (remarkably effective)], it is clear that the effective components of the preparations of Examples 1 to 6 are penetrated through human skin to increase blood flow volume remarkably.
(2) In the hair regrowth test of the preparations of Example 1 or Examples 2 to 6, hair regrowth took place in the area of 95% to 98% relative to the area that the preparation was applied, in contrast thereto, in the hair regrowth test of the preparations of Comparative Example 1 or 2, hair regrowth took place only in 5% relative to the area that the preparation was applied, the hair regrowth effect of the preparation of the present invention is far more excellent than that of the preparation of Comparative Example.
(3) The each evaluation of the preparations of Example 1 or Examples 2 to 6 by the hair restoration promoting test is ++ [Hair root resting phase ratio was reduced by at least 30% compared with Comparative Example 1 or 2], the preparations of Examples 1 to 6 exhibit an excellent hair restoration promoting effect.
(4) The each evaluation of the preparations of Example 1 or Examples 2 to 6 by the hair loss prevention test is ++ [Number of hair loss was decreased by at least 70], in contrast to the result of Comparative Example 1 or 2 (-) (hair loss prevention effect was not observed), the preparations of Examples 1 to 6 exhibit an excellent hair loss prevention effect.
   Additionally, in Examples 1 to 6, as the effective component, a prototype 1 (DNA salt degradation product), a prototype 2 (DNA degradation product), a prototype 3 (nucleoprotein degradation product), a prototype 4 (DNA and nucleoprotein degradation product), a prototype 5 (RNA degradation product) and a prototype 6 (mixture of prototype 2 and prototype 5) were used, when inplace of prototypes 1 to 6, a deoxyoligonucleotide and a deoxymononucleotide, an oligopeptide, an oligonucleotide or a mononucleotide which were separated and purified from the prototypes were used alone or in combination thereof, the same effects were observed as the cases where the prototypes 1 to 6 were used.

Namely, it has been recognized from the present examples that the preparations containing a deoxyoligonucleotide, a deoxymononucleotide, an oligopeptide, an oligonucleotide or a mononucleotide have the increase of blood flowvolume, hair regrowth effect, hair restoration promoting effect and hair loss prevention effect.

Hereinafter, a prescription example of hair care preparation using the above-described prototypes 1 to 6 is shown as Examples 7 to 9. Additionally, "prototype (s)" described herein means any one selected from prototypes 1 to 5 each alone or an equivalent mixture of prototype 2 and prototype 5 (prototype 6).
It has been recognized that any Example has the increase of blood flow volume, hair regrowth effect, hair restoration promoting effect and hair loss prevention effect.

### [Example 7] Liquid lotion preparation

**Table 3**

| Liquid lotion preparation: Prescription | |
|---|---|
| Blending component | Blending quantity (mass%) |
| 95% Ethanol | 80.0 |
| Prototype(s) | 1.0 |
| Nicotinic-acid amide | 0.5 |
| Cepharanthine | 0.01 |
| Dipropylene glycol | 2.0 |
| Disodium phosphate | 0.08 |
| Monopotassium phosphate | 0.02 |
| L-menthol | 0.3 |
| Fragrance | Appropriate amount |
| Purified water | Remainder |

### <Production method>

Prototype(s), nicotinic-acid amide, cepharanthine, dipropylene glycol, L-menthol and fragrance were dissolved in 95% ethanol.
Next, a solution that disodium phosphate and monopotassium phosphate were dissolved in purified water was added thereto and dissolved while stirring to prepare a liquid lotion preparation.

### [Example 8] Liquid tonic preparation

**Table 4**

| Liquid tonic preparation: Prescription | |
|---|---|
| Blending component | Blending quantity (mass%) |
| 95% Ethanol | 90.0 |
| Prototype(s) | 1.0 |
| Benzyl nicotinoate | 0.05 |
| Acetic acid α-DL-tocopherol | 0.5 |
| Propylene glycol | 1.0 |
| Polyethylene glycol 200 | 1.0 |
| Fragrance | Appropriate amount |
| Dye | Appropriate amount |
| Purified water | Remainder |

### <Production method>

Prototype(s), benzyl nicotinoate, acetic acid α-DL-tocopherol, propylene glycol, polyethylene glycol 200 and fragrance were added to 95% ethanol and dissolved.
Next, dye dissolved in purified water was added thereto and dissolved while stirring to prepare a liquid tonic preparation.

### [Example 9] Preparation in milky liquid form

**Table 5**

| Preparation in milky liquid form: Prescription | |
|---|---|
| Blending component | Blending quantity (mass%) |
| (A) phase | |
| Prototype(s) | 1.0 |
| Hardened caster oil ethylene oxide (60 mol) adduct | 2.0 |
| Glycerin | 10.0 |
| Dipropylene glycol | 10.0 |
| 1,3-Butylene glycol | 5.0 |
| Polyethylene glycol 1500 | 5.0 |

| (B) phase | |
|---|---|
| Cetyl isooctanate | 10.0 |
| Squalane | 5.0 |
| Vaseline | 2.0 |
| Propylparaben | 2.0 |

| (C) phase | |
|---|---|
| Carboxy vinyl polymer 1% aqueous solution | 30.0 |
| Sodium hexametaphosphate | 0.03 |
| Purified water | 8.0 |

| (D) phase | |
|---|---|
| Purified water | 4.5 |

| (E) phase | |
|---|---|
| Potassium hydrate | 0.12 |
| Purified water | Remainder |

### <Production method>

(A) phase and (B) phase were each heated at 70 °C and dissolved, mixed and subjected to a homogenizing mixer treatment to prepare a gel. (D) phase was slowly added to the gel and dispersed with a homogenizing mixer.
Next, (C) phase previously dissolved in this dispersed gel was added thereto, further, (E) phase previously dissolved was added and emulsified with a homogenizing mixer to prepare a preparation in milky liquid form.

### [Brief Description of the Drawings]

[FIG. 1]
   FIG. 1 is a diagram showing an analytical example of deoxyoligonucleotide from the treated product of DNA with nuclease (degradation product) by HPLC.

## Claims

1. A hair regrowth agent comprising a degradation product obtained by an enzymatic or hydrolytic treatment of a nucleoprotein and/or DNA, or a deoxyoligonucleotide, a deoxymononucleotide or an oligopeptide separated from the degradation product; or a degradation product obtained by an enzymatic or hydrolytic treatment of RNA, or an oligonucleotide or a mononucleotide separated from the degradation product; or a mixture of at least two members selected from the degradation product of a nucleoprotein and/or DNA, the degradation product of RNA, a deoxyoligonucleotide, a deoxymononucleotide, an oligopeptide, an oligonucleotide and a mononucleotide.

2. The hair regrowth agent according to claim 1, wherein the nucleoprotein and DNA are obtained from milt of fish.

3. The hair regrowth agent according to claim 2, wherein the fish is selected from the group consisting of salmon, trout, herring and cod.

4. The hair regrowth agent according to claim 1, wherein the yeast is beer yeast.

5. A hair restorer comprising a degradation product obtained by an enzymatic or hydrolytic treatment of a nucleoprotein and/or DNA, or a deoxyoligonucleotide, a deoxymononucleotide or an oligopeptide separated from the degradation product; or a degradation product obtained by an enzymatic or hydrolytic treatment of RNA, or an oligonucleotide or a mononucleotide separated from the degradation product; or a mixture of at least two members selected from the degradation product of a nucleoprotein and/or DNA, the degradation product of RNA, a deoxyoligonucleotide, a deoxymononucleotide, an oligopeptide, an oligonucleotide and a mononucleotide.

6. The hair restorer according to claim 5, wherein the nucleoprotein and DNA are obtained from milt of fish.

7. The hair restorer according to claim 6, wherein the fish is selected from the group consisting of salmon, trout, herring and cod.

8. The hair restorer according to claim 5, wherein the yeast is beer yeast.

9. An agent for preventing hair loss comprising a degradation product obtained by an enzymatic or hydrolytic treatment of a nucleoprotein and/or DNA, or a deoxyoligonucleotide, a deoxymononucleotide or an oligopeptide separated from the degradation product; or a degradation product obtained by an enzymatic or hydrolytic treatment of RNA, or an oligonucleotide or a mononucleotide separated from the degradation product; or a mixture of at least two members selected from the degradation product of a nucleoprotein and/or DNA, the degradation product of RNA, a deoxyoligonucleotide, a deoxymononucleotide, an oligopeptide, an oligonucleotide and a mononucleotide.

10. The agent for preventing hair loss according to claim 9, wherein the nucleoprotein and DNA are obtained from milt of fish.

11. The agent for preventing hair loss according to claim 10, wherein the fish is selected from the group consisting of salmon, trout, herring and cod.

12. The agent for preventing hair loss according to claim 9, wherein the yeast is beer yeast.
